# EUROPEAN PATENT SPECIFICATION

(11) **EP 2 846 935 B1**
(45) Date of publication and mention of the grant of the patent: **26.07.2017**
(21) Application number: 13720370.9
(22) Date of filing: 02.05.2013
(51) Int. Cl.: B08B 3/00, C07C 41/03, C07C 43/11, C11D 1/722, C11D 1/825, C11D 1/83

(54) **ALKOXYLATED ALCOHOLS AND THEIR USE IN FORMULATIONS FOR HARD SURFACE CLEANING**
ALKOXYLIERTE ALKOHOLE UND IHRE VERWENDUNG BEI FORMULIERUNGEN FÜR DIE REINIGUNG HARTER OBERFLÄCHEN
ALCOOLS ALCOXYLÉS ET LEUR UTILISATION DANS DES FORMULATIONS DE NETTOYAGE DE SURFACES DURES

(30) Priority: 10.05.2012 EP 12167493
(43) Date of publication of application: 18.03.2015
(73) Proprietor: BASF SE, 67056 Ludwigshafen am Rhein (DE)
(72) Inventor: REINOSO GARCIA, Marta, 69221 Dossenheim (DE); TROPSCH, Jürgen, 67354 Römerberg (DE); BAUER, Frederic, 67146 Deidesheim (DE); ÜNER, Ahmet, Ümraniye-Istanbul (TR)
(74) Representative: BASF IP Association
(86) International application number: PCT/EP2013/059078
(87) International publication number: WO 2013/167438

(56) References cited:
- EP-A1- 2 457 890
- EP-A2- 0 235 774
- WO-A1-85/02175
- WO-A1-2007/096292
- WO-A1-2010/070088
- WO-A1-2011/003904
- WO-A1-2011/130310
- US-A- 4 598 162
- US-A- 5 482 908
- US-A- 5 693 584

## Description

The present invention is directed towards the formulations, comprising at least one compound according to general formula (I) 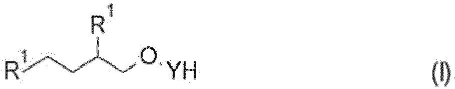
wherein R¹ is a CH(CH₃)₂-group,
YH is (AO)ₙ-H, with
AO being selected from -CH₂CH₂O- and CH₂CH(R²)O- and being different or identical in the case of n > 1

- R²: being different or identical and selected from C₁-C₁₆-alkyl, branched or linear,
- n: in the range of from 1 to 30,
and the polydispersity being in the range of from 1.0 to 1.5.

Furthermore, the present invention is directed towards the use of inventive formulations, comprising at least one compound according to formula (I), to compounds according to formula (I), and to a process for manufacturing inventive compounds and inventive formulations.

When cleaning surfaces such as hard surfaces or fibers with aqueous formulations several problems have to be solved. One task is to solubilize the dirt that is supposed to be removed and to keep it in the aqueous medium. Another task is to allow the aqueous medium to at all come into contact with the surface to be cleaned. Such a contact can be prevented, e. g., due to the high surface tension of water. Instead of a good wetting of the surface to be cleaned one observes bubbles of air sitting on such a surface and preventing the access of water to the respective surface.

It is desirable in many applications such as wetting or cleaning to reduce the surface tension of liquid aqueous formulations. For that purpose, usually wetting agents are being employed. Such wetting agents can be selected from surfactants which serve a dual purpose: to reduce the surface tension of the water and to prevent bubbles of air remaining on the surface.

In WO 2011/003904 a formulation has been disclosed that comprises an alkoxylated long-chain alcohol and an alkoxylated branched C₈-C₁₂-alcohol. They show good wetting and soil solubilizing behavior. However, in certain applications such as tiles that are exposed to heat, residual soil can be observed.

Tiles such as many other hard surfaces need thorough cleaning. Depending on where such tiles or other hard surfaces are located, e. g., in bath rooms, kitchens, hospitals, or the like, clean surfaces also mean good hygienic properties. Residual soil, to the contrary, can mean danger of non-hygienic environment.

Especially in cleaning of heavily soiled hard surfaces, such as heavily soiled tiles or heavily soiled metal surfaces, residual soil or residual grease can only be removed with great effort, if at all. Many surfactant formulations are not efficient enough to remove residual soil without severe mechanical effort.

It was therefore an objective to provide a formulation that allows less residual soil to stay on hard surfaces when employed in hard surface cleaning, especially when cleaning tiles. It was further more an objective to provide surfactants that leave less residual soil when applying them in hard surface or fiber cleaning. It was an objective to provide methods for making such surfactants and formulations, and it was an objective to provide a use for such formulations.

Accordingly, the formulations defined in the outset have been found.

Formulations according to the invention comprise at least one compound according to general formula (I) 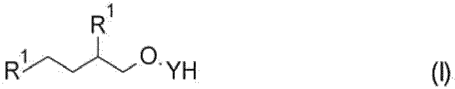 wherein the integers are defined as follows:
R¹ is iso-propyl, that is CH(CH₃)₂-groups.
YH is (AO)ₙ-H, with
AO being selected from -CH₂CH₂O- and -CH₂CH(R²)O- and being different or identical in the case of n > 1.

The group -CH₂CH₂O- can also be abbreviated as EO in the context of the present invention.

In one embodiment of the present invention, YH is selected from -[CH₂CH(R²)O]_{y}-[EO]ₓ, with x being selected from 1 to 30, preferably 1.5 to 15, even more preferably 2 to 10, and y being selected from zero to 5, preferably zero to 2 or 1 to 2.

In one embodiment of the present invention, YH is selected from -[EO]ₓ-[CH₂CH(R²)O]_{y}, with x being selected from 1 to 30, preferably 1.5 to 15, even more preferably 2 to 10, and y being selected from zero to 5, preferably zero to 2.

In one embodiment of the present invention, YH is selected from -[EO]ₓ-[CH₂CH(R²)O]_{y}, with x being selected from 1 to 30, preferably 1.5 to 15, even more preferably 2 to 10, and y being selected from zero to 5, preferably zero to 2.

The integers x and y are average values in the context of the present invention, preferably number average.

In one embodiment of the present invention, YH is selected from (AO)ₙ, AO being identical and selected from -CH₂CH₂O- and -CH₂CH(R²)O-, preferably EO.
- R²: are different or identical and selected from C₁-C₁₆-alkyl, branched or linear, preferably linear, more preferably n-propyl, ethyl and even more preferably methyl.
- n: is in the range of from 1 to 30. The integer n is an average value in the context of the present invention, preferable number average values for n are 1.5 to 15, even more preferred 2 to 10.

The polydispersity (M_{w}/Mₙ) of compound according to general formula (I) is in the range of from 1.0 to 1.5.

In one embodiment of the present invention, inventive formulations contain in the range of from 0.01 to 95 % by weight compound of general formula (I), percentages being based on total weight of inventive formulations.

In one embodiment of the present invention, inventive formulations contain in the range of from 2 to 50 % by weight, preferably 3 to 20 % by weight of compound of general formula (I). Such inventive formulations can be used as, e.g., bathroom cleaners, industrial hard surface cleaners, and kitchen cleaners.

In one embodiment of the present invention, inventive formulations contain in the range of from 0.01 to 15 % by weight, preferably 0.02 to 7.5 % by weight of compound of general formula (I). Such inventive formulations can be used as, e.g., glass cleaners, and especially window cleaners.

In one embodiment of the present invention, inventive formulations can also contain at least one compound according to the general formula (IV) 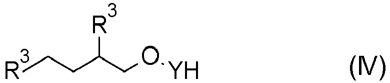 wherein the integers are defined as follows:
R³ are selected from non-branched C₃-alkyl groups, in other words, compounds of general formulae (I) and (IV) in the respective inventive formulation being isomers.

In one embodiment of the present invention, inventive formulations can contain at least one compound according to general formulae (V a to (V c)) 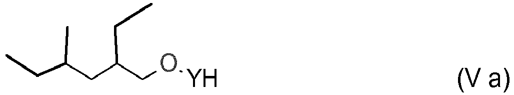 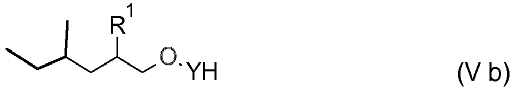 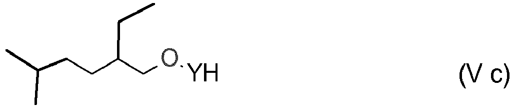 wherein the integers are defined as above.

In one embodiment of the present invention, inventive formulations contain
(A) a total of 90.5 to 99 % by weight of at least one compound according to general formula (I),
(B) a total of 0.5 to 10 % by weight of at least one compound according to formulae (V a) to (V c).

Compounds according to general formulae (I) and (IV) can be distinguished, e. g., by standard analytical means such as HPLC or ¹³C NMR spectroscopy or two-dimensional ¹H NMR spectroscopy. Preferred are coupling methods (COSY, DEPT, INADEQUATE), followed by a quantification via ¹³C NMR with relaxation reagents. However, other NMR methods or GC-MS (gas chromatography coupled with mass spectrometry) methods are also possible Inventive formulations can be solid, liquid or in the form of slurries. Preferably, inventive formulations are selected from liquid and solid formulations. In one embodiment, inventive formulations are aqueous, preferably liquid aqueous formulations.

In one embodiment of the present invention, inventive formulations can contain 0.1 to 90 % by weight of water, based on total of the respective formulation.

In one embodiment of the present invention, inventive formulations have a pH value in the range of from zero to 14, preferably from 2.5 to 11. The pH value can be chosen according to the type of hard surface and the specific application. It is, e.g., preferred to select a pH value in the range of from 1 or less up to 2.5 for bathroom or toilet cleaners. It is furthermore preferred to select a pH value in the range of from 4 to 10 for dishwashing or floor cleaners.

In one embodiment of the present invention, inventive formulations contain at least one active ingredient. Active ingredients can be selected from soaps, anionic surfactants, such as LAS (linear alkylbenzenesulfonate) or paraffine sulfonates or FAS (fatty alcohol sulfates) or FAES (fatty alcohol ether sulfates), furthermore acids, such as phosphoric acid, amidosulfonic acid, citric acid, lactic acid, acetic acid, other organic and inorganic acids, furthermore organic solvents, such as butyl glycol, n-butoxypropanol, especially 1-butoxy-2-propanol, ethylene glycol, propylene glycol, glycerine, ethanol, monoethanolamine, and isopropanol.

In one embodiment of the present invention, inventive formulations comprise at least one organic acid, selected from acetic acid, citric acid, and methanesulfonic acid.

In one embodiment of the present invention, inventive formulations contain at least one or more active ingredients selected from non-ionic surfactants which are different from compounds of formulae (I) and (IV). Examples of suitable non-ionic surfactants are alkoxylated n-C₁₂-C₂₀-fatty alcohols, such as n-C₁₀-C₂₀-alkyl(EO)ₘOH with m being in the range of from 5 to 100, furthermore block copolymers of ethylene oxide and propylene oxide, such as poly-EO-poly-PO-poly-EO with M_{w} in the range of from 3,000 to 5,000 g/mol PO content of from 20 to 50% by mass, furthermore alkyl polyglycosides, preferably branched C₈-C₁₀-alkyl polyglucosides, especially C₈-C₁₀-alkyl polyglucosides with a branching in 2-position of the respective C₈-C₁₀-alkyl group.

In one embodiment of the present invention, inventive formulations can be used as bath cleaners, as sanitary cleaners, as kitchen cleaners, as toilet cleaners, as toilet bowl cleaners, as sanitary descalers, as all-purpose household cleaners, as all-purpose household cleaner concentrates, as metal degreasers, as all purpose-household spray cleaners, as hand dish cleaners, as automatic dishwashing agents, or floor cleaners, as hand cleaners.

In one embodiment of the present invention, inventive formulations can contain at least one biocide or preservative, such as benzalkonium chlorides.

In another embodiment of the present invention, inventive formulations can be used as laundry detergents.

In one embodiment of the present invention, inventive formulations can contain one or more active ingredients selected from inorganic builders such as phosphates, such as triphosphates. Phosphate-free formulations according to the present invention are preferred. In the context of the present invention, the term "phosphate-free" refers to formulations with 0.5 % by weight of phosphate maximum, based on the total solids content and measured by gravimetric methods, and phosphate-free formulations can contain a minimum of 50 ppm (weight) phosphate or less.

Examples of preferred inorganic builders are silicates, silicates, carbonates, and alumosilicates. Silicates and alumosilicates can be selected from crystalline and amorphous materials.

In one embodiment of the present invention, inorganic builders are selected from crystalline alumosilicates with ion-exchanging properties, such as, in particular, zeolites. Various types of zeolites are suitable, in particular zeolites A, X, B, P, MAP and HS in their Na form or in forms in which Na is partially replaced by cations such as Li⁺, K⁺, Ca²⁺, Mg²⁺ or ammonium.

Suitable crystalline silicates are, for example, disilicates and sheet silicates. Crystalline silicates can be used in the form of their alkali metal, alkaline earth metal or ammonium salts, preferably as Na, Li and Mg silicates.

Amorphous silicates, such as, for example, sodium metasilicate, which has a polymeric structure, or Britesil^{®} H20 (manufacturer: Akzo) can be selected.

Suitable inorganic builders based on carbonate are carbonates and hydrogencarbonates. Carbonates and hydrogencarbonates can be used in the form of their alkali metal, alkaline earth metal or ammonium salts. Preferably, Na, Li and Mg carbonates or hydrogencarbonates, in particular sodium carbonate and/or sodium hydrogencarbonate, can be selected. Other suitable inorganic builders are sodium sulphate and sodium citrate.

In one embodiment of the present invention, inventive formulations can contain at least one organic complexing agent (organic cobuilders) such as EDTA (N,N,N',N'-ethylenediaminetetraacetic acid), NTA (N,N,N-nitrilotriacetic acid), MGDA (2-methylglycine-N,N-diacetic acid), GLDA (glutamic acid N,N-diacetic acid), and phosphonates such as 2-phosphono-1,2,4-butanetricarboxylic acid, aminotri(methylenephosphonic acid), 1-hydroxyethylene(1,1-diphosphonic acid) (HEDP), ethylenediaminetetramethylenephosphonic acid, hexamethylenediaminetetramethylenephosphonic acid and diethylenetriaminepentamethylenephosphonic acid and in each case the respective alkali metal salts, especially the respective sodium salts. Preferred are the sodium salts of HEDP, of GLDA and of MGDA.

In one embodiment of the present invention, inventive formulations can contain one or more active ingredients selected from organic polymers, such as polyacrylates and copolymers of maleic acid-acrylic acid.

In one embodiment of the present invention, inventive formulations can contain one or more active ingredients selected from alkali donors, such as hydroxides, silicates, carbonates.

In one embodiment of the present invention, inventive formulations can contain one or more further ingredients such as perfume oils, oxidizing agents and bleaching agents, such as perborates, peracids or trichloroisocyanuric acid, Na or K dichloroisocyanurates, and enzymes.

Most preferred enzymes include lipases, amylases, cellulases and proteases. In addition, it is also possible, for example, to use esterases, pectinases, lactases and peroxidases.

Enzyme(s) may be deposited on a carrier substance or be encapsulated in order to protect them from premature decomposition.

In one embodiment of the present invention, inventive formulations can contain one or more active ingredients such as graying inhibitors and soil release polymers.

Examples of suitable soil release polymers and/or graying inhibitors are:
Polyesters of polyethylene oxides and ethylene glycol and/or propylene glycol as diol component(s) with aromatic dicarboxylic acids or combinations of aromatic and aliphatic dicarboxylic acids as acid component(s),
polyesters of aromatic dicarboxylic acids or combinations of aromatic and aliphatic dicarboxylic acids as acid component(s) with di- or polyhydric aliphatic alcohols as diol component(s), in particular with polyethylene oxide, said polyesters being capped with polyethoxylated C₁-C₁₀-alkanols.

Further examples of suitable soil release polymers are amphiphilic copolymers, especially graft copolymers of vinyl esters and/or acrylic esters onto polyalkylene oxides. Further examples are modified celluloses such as, for example, methylcellulose, hydroxypropylcellulose and carboxymethylcellulose.

In one embodiment of the present invention, inventive formulations can contain one or more active ingredients selected from dye transfer inhibitors, for example homopolymers and copolymers of vinylpyrrolidone, of vinylimidazole, of vinyloxazolidone or of 4-vinylpyridine N-oxide, each having average molar masses M_{w} of from 15,000 to 100,000 g/mol, and cross-linked finely divided polymers based on the above monomers;

In one embodiment of the present invention, inventive formulations contain 0.1 to 50% by weight, preferably 1 to 20 % by weight organic complexing agent, based on the total solids content of the respective inventive formulation.

In one embodiment of the present invention, inventive formulations contain 0.1 to 80% by weight, preferably 5 to 55 % by weight anionic surfactant, based on the total solids content of the respective inventive formulation.

In one embodiment of the present invention, inventive formulations can contain one or more active ingredients selected from defoamers. Examples of suitable defoamers are silicon oils, especially dimethyl polysiloxanes which are liquid at room temperature, without or with silica particles, furthermore microcrystalline waxes and glycerides of fatty acids.

In one embodiment of the present invention, inventive formulations do not contain any defoamer which shall mean in the context of the present invention that said inventive formulations comprise less than 0.1 % by weight of silicon oils and less than 0.1 % by weight of glycerides of fatty acids and less than 0.1 % by weight of microcrystalline waxes, referring to the total solids content of the respective inventive formulation, and in the extreme, no measureable amounts of silicon oils glycerides of fatty acids at all.

It has been found that formulations disclosed in WO 2011/003904 can produce a certain amount of foam that is disadvantageous in various applications. The foam can transport soil to places not being easy to access and there it remains, together with the residual foam. Said residual foam may be due to incomplete rinsing.

Inventive formulations show an extremely good performance when using them for cleaning hard surfaces, in particular tiles.

A further aspect of the present invention is the use of inventive formulations for hard surface or fiber cleaning. A further aspect of the present invention is a method for cleaning hard surfaces under application of inventive formulations, hereinafter also briefly referred to as inventive cleaning method. Hard surfaces as used in the context with the present invention are defined as surfaces of water-insoluble and - preferably - non-swellable materials. Hard surfaces as used in the context of the present invention preferably also exhibit resistance against manual destruction such as scratching with fingernails. Preferably, they have a Mohs hardness of 3 or more. Examples of hard surfaces are glassware, tiles, stone, china, enamel, concrete, leather, steel, other metals such as iron or aluminum, furthermore wood, plastic, in particular melamine resins, polyethylene, polypropylene, PMMA, polycarbonates, polyesters such as PET, furthermore polystyrene and PVC, and furthermore, silicon (wafers) surfaces. Particularly advantageous are inventive formulations when used for cleaning hard surfaces that are at least part of structured objects. In the context, such structured objects refer to objects having, e. g. convex or concave elements, notches, furrows, corners, or elevations like bumps.

Fibers as used in the context with the present invention can be of synthetic or natural origin. Examples of fibers of natural origin are cotton and wool. Examples of fibers of synthetic origin are polyurethane fibers such as Spandex® or Lycra®, polyester fibers, polyamide fibers, and glass wool. Other examples are biopolymer fibers such as viscose, and technical fibers such as GoreTex®. Fibers may be single fibers or parts of textiles such as knitwear, wovens, or nonwovens.

In order to perform the inventive cleaning method inventive formulations are being applied. Preferably, inventive formulations are applied in their embodiments as aqueous formulations, comprising, e. g., 10 to 99.9 % by weight water. Inventive formulations can be dispersions, solutions, gels, or solid blocks, emulsions including microemulsions, and foams, preferred are solutions. They can be used in highly diluted form, such as 1:10 up to 1:50.

In order to perform the inventive cleaning method, any hard surface or fiber or arrangement of fibers can be contacted (brought into contact) with an inventive formulation.

When contacting hard surfaces with inventive formulations, inventive formulations can be applied at ambient temperature. In a further embodiment, inventive formulations can be used at elevated temperatures, such as 30 to 85°C, for examples by using an inventive formulation that has a temperature of 30 to 85°C, or by applying an inventive formulation to a preheated hard surface, e. g., preheated to 30 to 85°C.

In one embodiment, it is possible to apply an inventive formulation to a hard surface under normal pressure. In a further embodiment, it is possible to apply inventive formulation to a hard surface under pressure, e. g., by use of a high-pressure cleaner or a pressure washer.

In one embodiment of the present invention, application duration of inventive formulation can be in the range of from one second up to 24 hours, preferably in the range of 30 min to 5 hours in the case of fiber cleaning and preferably one second up to 1 hour in cases such as floor cleaning, kitchen cleaning or bathroom cleaning.

Hard surface cleaning in the context of the present invention can include removing heavy soiling, removing slight soiling and removing dust, even removing small quantities of dust.

Examples of soiling to be removed are not limited to dust and soil but can be soot, hydrocarbons, e.g., oil, engine oil, furthermore residues from food, drinks, body fluids such as blood or excrements, furthermore complex natural mixtures such as grease, and complex synthetic mixtures such as paints, coatings, and pigment containing grease.

The contacting of the hard surface with inventive formulation can be performed once or repeatedly, for example twice or three times.

After having performed the contacting the hard surface with inventive formulation the remaining inventive formulation containing soil or dust will be removed. Such removal can be effected by removal of the object with the now clean hard surface from the respective inventive formulation or vice versa, and it can be supported by one or more rinsing step(s).

After having performed the inventive cleaning method, the object with the now-clean hard surface can be dried. Drying can be effected at room temperature or at elevated temperature such as, e.g., 35 to 95°C. Drying can be performed in a drying oven, in a tumbler (especially with fibers and with fabrics), or in a stream of air having room temperature or elevated temperature such as 35 to 95°C. Freeze-drying is another option.

By performing the inventive cleaning method, hard surfaces can be cleaned very well. In particular, objects with structured hard surfaces can be cleaned well.

A further aspect of the present invention is a process of making inventive formulations, briefly also being referred to as inventive process. The inventive process comprises the step of mixing at least one compound of general formula (I) and optionally with at least one active ingredient, preferably selected from organic complexing agents, anionic surfactants and organic acids, selected from acetic acid, citric acid, and methanesulfonic acid. Mixing can be achieved, e. g., by stirring the ingredients into water. After mixing, the water can be removed in whole or in part by, e.g., spray drying. Mixing can be achieved, e. g., at ambient temperature or at slightly elevated temperature, for example in the range of from 30 to 45°C.

A further aspect of the present invention is a compound of general formula (I) 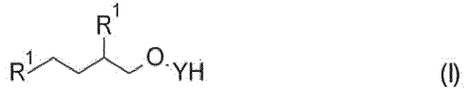
wherein R¹ is a CH(CH₃)₂-group,
YH is (AO)ₙ-H, with AO being selected from -CH₂CH₂O- and -CH₂CH(R²)O- and being different or identical in the case of n > 1,

- R²: being different or identical and selected from C₁-C₁₆-alkyl, branched or linear,
- n: in the range of from 1 to 30,
and the polydispersity being in the range of from 1.0 to 1.5.

The polydispersity (M_{w}/Mₙ) of inventive compound according to general formula (I) is in the range of from 1.0 to 1.5.

The integers and their preferred embodiments are defined in more detail above.

Inventive compounds can advantageously be used for making inventive formulations.

Inventive compounds may contain compounds of general formula (III) as an impurity, said impurity also being referred to as residual alcohols. In a preferred embodiment, inventive mixtures contain in the range of from 100 ppm to 1 % b weight of residual alcohols.

A further aspect of the present invention is a process for making an inventive mixture, briefly also referred to as inventive synthesis, comprising the step of reacting the corresponding alcohol according to formula (III) 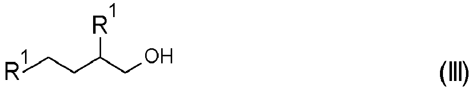 with R¹ being defined as above,
with at least one alkylene oxide selected from ethylene oxide and alkylene oxides according to formula (II) 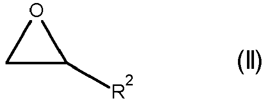
- R²: being different or identical and selected from C₁-C₁₆-alkyl, branched or linear,
in the presence of at least one catalyst, selected from double-metal cyanides.

The integers are defined as above.

Compounds of general formula (III) are known per se. They can be obtained by, e. g., Guerbet reaction of alcohols of general Formula R¹-CH₂-CH₂-OH, with R¹ being defined as above.

Double-metal cyanides, hereinafter also referred to as double-metal cyanide compounds or DMC compounds, usually comprise at least two different metals, at least one of them being selected from transition metals and the other one being selected from transition metals and alkali earth metals, and furthermore cyanide counterions. Particularly suitable catalysts for the alkoxylation are double-metal cyanide compounds which contain zinc, cobalt or iron or two thereof. Berlin blue, for example, is particularly suitable.

Preference is given to using crystalline DMC compounds. In a preferred embodiment, a crystalline DMC compound of the Zn-Co type which comprises zinc acetate as further metal salt component is used as catalyst. Such compounds crystallize in monoclinic structure and have a platelet-like habit. Such compounds are described, for example, in WO 00/74845 and in WO 01/64772.

Examples of double-metal cyanide compounds suitable as catalyst are described in WO 2003/091192.

Double-metal cyanide compounds can be used as powder, paste or suspension or be moulded to give a moulding, be introduced into mouldings, foams or the like or be applied to mouldings, foams or the like.

The double-metal cyanide compound concentration used for the inventive synthesis, based on the alcohol according to general formula (III), is preferably less than 2000 ppm (i.e. mg of catalyst per kg of product), more preferably less than 1000 ppm, in particular less than 500 ppm, particularly preferably less than 100 ppm, for example less than 50 ppm or 35 ppm, particularly preferably less than 25 ppm; ppm referring to mass-ppm (parts per million) of compound of general formula (III).

In one embodiment of the present invention, the inventive synthesis is carried out at temperatures in the range of from 90 to 240°C, preferably from 120 to 180°C, in a closed vessel.

In the context of the present invention, ethylene oxide or alkylene oxide of general formula (III) or a mixture of different alkylene oxides of general formula (III) or a mixture of ethylene oxide and alkylene oxide of general formula (III) can also generally be referred to as "alkylene oxide(s)".

In one embodiment of the present invention, alkylene oxide(s) is/are introduced into a mixture of compound of general formula (III) and double-metal cyanide compound under the vapour pressure of the alkylene oxide(s) prevailing at the selected reaction temperature. Alkylene oxide(s) can be introduced in pure form or, as an alternative, be diluted up to about 30 to 60% by volume with an inert gas such as a rare gas or nitrogen. This affords additional safety against explosion-like polyaddition of the alkylene oxide.

In case several alkylene oxides are being introduced polyether chains will be formed in which the different alkylene oxide units are distributed virtually randomly in compounds of general formula (I). Variations in the distribution of the units along the polyether chain can arise due to differing reaction rates of the alkylene oxides. Variations in the distribution of the units along the polyether chain can be achieved arbitrarily by continuously introducing an alkylene oxide mixture of program-controlled composition as well. In case different alkylene oxides are reacted subsequently, then polyether chains with a block-type distribution of the alkylene oxide units are obtained. In another embodiment with different alkylene oxides being reacted, addition of one alkylene oxide is being started before the other alkylene oxide has been completely reacted.

Preferred inventive mixtures can be obtained by reacting compound of general formula (III) firstly with propylene oxide and then with ethylene oxide under conditions indicated above.

Further preferred inventive mixtures can be obtained by reacting compound of general formula (III) solely with ethylene oxide.

In one embodiment of the present invention, the inventive synthesis is carried out without a diluent or solvent.

In one embodiment of the present invention, the inventive synthesis can be carried out using a solvent. Suitable solvents are, for example, N,N-dimethyl formamide, toluene, xylene, and mixtures of toluene and xylene, or solvent naphtha.

In one embodiment of the present invention the inventive synthesis is carried out in a way that a reaction with alkoxide of general formula (II), especially with propylene oxide, is carried out first, followed by subsequent ethoxylation. In said embodiment, the content of residual alcohol in the alkoxylates can be reduced, it being observed that propylene oxide is reacted more evenly with compound of general formula (III).

In many embodiments, the conversion with alkylene oxide(s) is incomplete with respect to alkylene oxide(s). In one embodiment of the present invention, alkylene oxide(s) is/are reacted in a molar excess of at least 1.5 · n, referring to the total of compound according to general formula (III).

In one embodiment of the present invention, the inventive synthesis is carried out in the presence of at least one double-metal cyanide selected from hexacyano cobaltates.

In one embodiment of the present invention, the inventive synthesis is carried out in the presence of at least one double-metal cyanide selected from compounds according to general formula (VI)

M¹ₐ[M²(CN)_{b}(A)_{c}]_{d}·f M¹_{g}Xₘ·h(H₂O)·eL·kP (VI),

in which the integers are defined as follows:
M¹ is at least one metal ion chosen from the group consisting of Zn²⁺, Fe²⁺, Fe³⁺, Co³⁺, Ni²⁺, Mn²⁺, Co²⁺, Sn2⁺, Pb²⁺, Mo⁴⁺, Mo⁶⁺, Al³⁺, V⁴⁺, V⁵⁺, Sr²⁺, W⁴⁺, W⁶⁺, Cr²⁺, Cr³⁺, Cd²⁺, Hg²⁺, Pd²⁺, Pt²⁺, V²⁺, Mg²⁺, Ca²⁺, Ba²⁺, Cu²⁺, La³⁺, Ce³⁺, Ce⁴⁺, Eu³⁺, Ti³⁺, Ti⁴⁺, Ag⁺, Rh²⁺, Rh³⁺, Ru²⁺, Ru³⁺,
M² is at least one metal ion chosen from the group consisting of Fe²⁺, Fe³⁺, Co²⁺, Co³⁺, Mn²⁺, Mn³⁺, V⁴⁺, V⁵⁺, Cr²⁺, Cr³⁺, Rh³⁺, Ru²⁺, Ir³⁺,
and in a way that M¹ and M² are not identical,
A and X, independently of one another, are anions selected from the group consisting of halide, hydroxide, sulfate, carbonate, cyanide, thiocyanate, isocyanate, cyanate, carboxylate, oxalate, nitrate, nitrosyl, hydrogensulfate, phosphate, dihydrogenphosphate, hydrogenphosphate or hydrogencarbonate,
L is a ligand chosen from the group consisting of alcohols, aldehydes, ketones, ethers, polyethers, esters, polyesters, polycarbonate, ureas, amides, primary, secondary and tertiary amines, ligands with pyridine nitrogen, nitriles, sulfides, phosphides, phosphites, phosphanes, phosphonates and phosphates,
k is an integer greater than or equal to zero, and up to 6. The integer k can be a whole number or a fraction.
P is an organic additive,
a, b, c, d, g and n are chosen such that the electroneutrality of the compound (VI) is ensured, where each f and c may be 0,
e is the number of ligand molecules, for example a fraction or an integer greater than zero, or zero,
f and h, independently of one another, are fractions or integers greater than zero, or zero.

In one embodiment, the upper limits of e, f, and h are 6.

In one embodiment, organic additive P is selected from polyethers, polyesters, polycarbonates, polyalkylene glycol sorbitan esters, polyalkylene glycol glycidyl ethers, polyacrylamide, poly(acrylamide-co-acrylic acid), polyacrylic acid, poly(acrylamide-co-maleic acid), polyacrylonitrile, polyalkyl acrylates, polyalkyl methacrylates, polyvinyl methyl ether, polyvinyl ethyl ether, polyvinyl acetate, polyvinyl alcohol, poly-N-vinylpyrrolidone, poly(N-vinylpyrrolidone-co-acrylic acid), polyvinyl methyl ketone, poly(4-vinylphenol), poly(acrylic acid-co-styrene), oxazoline polymers, polyalkyleneimines, maleic acid and maleic anhydride copolymers, hydroxyethylcellulose, polyacetates, ionic surface-active and interface-active compounds, bile acid or salts thereof, esters or amides, carboxylic esters of polyhydric alcohols and glycosides.

Double-metal cyanide compounds may be crystalline or amorphous. When k is zero, crystalline double-metal cyanide compounds are preferred. When k is greater than zero, crystalline, partially crystalline and also substantially amorphous catalysts are preferred.

There are various preferred embodiments of the modified catalysts. A preferred embodiment covers catalysts of the formula (VI) in which k is greater than zero. The preferred catalyst then comprises at least one double-metal cyanide compound, at least one organic ligand and at least one organic additive P.

In another preferred embodiment of the inventive synthesis, k is zero, optionally e is also zero and X is exclusively a carboxylate, preferably formate, acetate and propionate. In this embodiment, preference is given to crystalline double-metal cyanide catalysts. Other preferred embodiments are double-metal cyanide catalysts as described in WO 00/74845, which are crystalline and platelet-like.

In another preferred embodiment of the catalysts, f, e and k do not equal zero. Such embodiments refer to double-metal cyanide catalysts which contain L, preferably in amounts of from 0.5 to 30% by weight based on compound of general formula (VI) and an organic additive P (generally in amounts of from 5 to 80% by weight), as described in WO 98/06312. Such catalysts can either be prepared with vigorous stirring (24,000 rpm using Turrax) or with stirring, as described in US 5,158,922.

In one embodiment of the inventive synthesis double-metal cyanide compounds can be are prepared by combining a metal salt solution with a cyanometallate solution, which may optionally contain both a ligand L and also an organic additive P. Subsequently, the organic ligand and optionally the organic additive are added. In a preferred embodiment of the catalyst preparation, an inactive double-metal cyanide compound is firstly prepared, and this is then converted into its active phase by recrystallization, as described in WO 01/64772.

DMC compounds suitable as catalysts may, in principle, be prepared by all ways known to the person skilled in the art. For example, the DMC compounds can be prepared by direct precipitation, incipient wetness method, by preparing a precursor phase and subsequent recrystallization.

It is possible to work up the product so obtained. However, in most cases the product so obtained has sufficient purity and can be used without further work-up. In other embodiments, the reaction mixture can be filtered, e.g., over cellulose filter material, or degassed under reduced pressure. In such cases, reduced pressure may mean, e.g., 10 to 50 mbar at a temperature in the range of from 30 to 100°C.

By performing the inventive synthesis, inventive compounds can be obtained in excellent yields.

The present invention is further illustrated by working examples.

The following catalyst was used: Zn₃[Co(CN)₆]2 ·ZnCl₂·H₂O, commercially available as Arcol Catalyst 3.
rpm: rounds per minute.

### I. Synthesis of inventive compounds

### I.1 Synthesis of inventive compound (I.1)

A 2-liter autoclave with anchor stirrer was charged with 189.8 g (1.2 mol) of 2-isopropyl-5-methylhexan-1-ol and 0.61 g of catalyst. The resultant mixture was heated under stirring (100 rpm) to 80°C, and flushed 3 times with dry nitrogen. A pressure of 1.5 bar was set by introducing dry nitrogen. The temperature was raised to 130°C. Then, a total of 422 g (9.6 moles) of ethylene oxide were introduced, distributed in three portions.

In a first portion, 50 g of ethylene oxide were introduced.

After 10 minutes, another 50 g of ethylene oxide were introduced. The stirrer was then set to 200 rpm.

Within the following 3 hours, another 322 g of ethylene oxide were introduced.

The mixture was stirred for another 6 hours at 130°C. Then, the mixture was cooled down to 80°C and flushed three times with nitrogen. It was then degassed at 100°C/20 mbar. Then, the mixture was filtered over a cellulose filter.

The resultant inventive compound (1.1) was a white solid at ambient temperature. The yield was almost quantitative. The residual alcohol content was 0.5% by weight.

Hydroxyl number., determined according to DIN 53240: 112.6 mg KOH/g

The polydispersity was determined by gel permeation chromatography (GPC), with tetrahydrofuran (THF) as eluent.
M_{w}/Mₙ: 1.16

### I.2 Synthesis of inventive compound (1.2)

A 2-liter autoclave with anchor stirrer was charged with 189.8 g (1.2 mol) of 2-isopropyl-5-methylhexan-1-ol and 0.61 g of catalyst. The resultant mixture was heated under stirring (100 rpm) to 80°C, and flushed 3 times with dry nitrogen. A pressure of 1.5 bar was set by introducing dry nitrogen. The temperature was raised to 130°C. Then, 104.6 g (1.8 mol) of propylene oxide and a total of 422 g (9.6 moles) of ethylene oxide were introduced subsequently, the ethylene oxide being distributed in two portions. At first, the propylene was introduced. Then a first portion, 50 g, of ethylene oxide were introduced.

Within the following 3 hours, another 372 g of ethylene oxide were introduced.

The mixture was stirred for another 6 hours at 130°C. Then, the mixture was cooled down to 80°C and flushed three times with nitrogen. It was then degassed at 100°C/20 mbar. Then, the mixture was filtered over a cellulose filter.

The resultant inventive compound (1.2) was a white solid at ambient temperature. The yield was almost quantitative. The residual alcohol content was 0.5% by weight.

Hydroxyl number., determined according to DIN 53240: 96.0 mg KOH/g
M_{w}/Mₙ: 1.16

### II. Manufacture of inventive formulations

### II.1 Manufacture of formulations usable as kitchen cleaners

The order of addition was as follows: First, a bucket was charged with demineralized water. Then, under stirring, compound (I.1) or (1.2) was added - or, in case of comparative examples, surfactants (C-VII.1) or (C-VII.2). Then, further ingredients according to table 1 were added, the last one being 32 % by weight aqueous NaOH to adjust the pH value to 12. The respective kitchen cleaners were obtained as clear, homogeneous liquids. 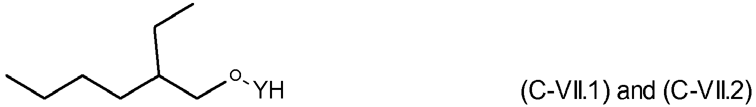
(C-VII.1): Y = (PO)_{1.5}(EO)₈
(C-VII.2): Y = (EO)₈

The molecular weights of (C-VII.1) and (C-VII.2) were determined by GPC in the same way as with inventive compounds (I.1) and (I.2).

**Table 1: Kitchen Cleaners according to the invention and Comparative Kitchen Cleaners**

| Component | IF.1 | IF.2 | C-F.3 | C-F.4 |
|---|---|---|---|---|
| (I.1) | 3 | - | - | - |
| (I-2) | - | 3 | - | - |
| (C-VII.1) | - | - | 3 | - |
| (C-VII.2) | - | - | - | 3 |
| Isopropanol | 1 | 1 | 1 | 1 |
| Sodium metasilicate | 0.5 | 0.5 | 0.5 | 0.5 |
| 1-butoxy-2-propanol | 0.5 | 0.5 | 0.5 | 0.5 |
| monoethanolamine | 1 | 1 | 1 | 1 |
| 32 % wt NaOH | 0.25 | 0.25 | 0.25 | 0.25 |
| Demineralized water | 93.75 | 93.75 | 93.75 | 93.75 |

| | | | | |
|---|---|---|---|---|
| All quantities in g/100 g | | | | |

### II.2 Manufacture of formulations usable as all-purpose cleaners

The order of addition was as follows: First, a bucket was charged with demineralized water. Then, under stirring, compound (I.1) or (I.2) was added - or, in case of comparative examples, surfactants (C-VII.1) or (C-VII.2). Then, further ingredients according to table 2 were added, the last one being citric acid to adjust the pH value to 6.5. The respective all-purpose cleaners were obtained as clear, homogeneous liquids.

**Table 2: All-purpose Cleaners according to the invention and Comparative All-purpose Cleaners**

| Component | IF.5 | IF.6 | C-F.7 | C-F.8 |
|---|---|---|---|---|
| (I.1) | 3 | - | - | - |
| (I-2) | - | 3 | - | - |
| (C-VII.1) | - | - | 3 | - |
| (C-VII.2) | - | - | - | 3 |
| Isopropanol | 5 | 5 | 5 | 5 |
| preservative | 0.1 | 0.1 | 0.1 | 0.1 |
| Citric acid | 0.1 | 0.1 | 0.1 | 0.1 |
| Demineralized water | 91.8 | 91.8 | 91.8 | 91.8 |

| | | | | |
|---|---|---|---|---|
| All quantities in g/100 g | | | | |

### II.2 Manufacture of formulations usable as bathroom cleaners

The order of addition was as follows: First, a bucket was charged with demineralized water. Then, under stirring, compound (I.1) or (I-2) was added - or, in case of comparative examples, surfactants (C-VII.1) or (C-VII.2). Then, further ingredients according to table 3 were added, the last one being citric acid to adjust the pH value to 3. The respective bathroom cleaners were obtained as clear, homogeneous liquids.

**Table 3: Bathroom Cleaners according to the invention and Comparative Bathroom Cleaners**

| Component | IF.9 | IF.10 | C-F.11 | C-F.12 |
|---|---|---|---|---|
| (I.1) | 3 | - | - | - |
| (I-2) | - | 3 | - | - |
| (C-VII.1) | - | - | 3 | - |
| (C-VII.2) | - | - | - | 3 |
| Isopropanol | 5 | 5 | 5 | 5 |
| Citric acid | 2 | 2 | 2 | 2 |
| Demineralized water | 90 | 90 | 90 | 90 |

| | | | | |
|---|---|---|---|---|
| All quantities in g/100 g | | | | |

### III. Hard surface cleaning tests

### III.1 Kitchen cleaning

Kitchen tiles were tiles with a color acceptable in a kitchen.

A standard soil was prepared, by mixing 25 g butter, 25 g lard, 25 g margarine, 12.5 g ketchup, and 12.5 mustard in a beaker on a water bath at 40 to 45°C under continuous stirring. Then, a roller was dipped into the soil so obtained and about 3 g of the soil were applied to a precleaned (ethanol) kitchen tiles. Then, the standard soil was evenly distributed on the kitchen tiles and the kitchen tiles then dried in an oven (180°C, 2 hours). They were then allowed to cool to ambient temperature. Soiled tiles were obtained.

Kitchen cleaners according to table 1 were filled into 4 different trigger dispensers. Each two soiled tiles were selected, and each of them sprayed with kitchen cleaner: the left half of the surface of the first soiled tile was sprayed with 3.5 g of IF.1 and the right half with C-F.4. The left half of the surface of the second soiled tile was sprayed with 3.5 g of IF.2 and the right half with C-F.3. The kitchen cleaners were allowed to rest for 2 minutes. Then, the different parts were wiped with cellulose type wet sponges without using force. Then, the kitchen cleaners were removed by rinsing with cold tap water.

The experiment was repeated 4 times, with fresh soiled tiles.

The removal of the soil was assessed visually.

In all cases, removal of the standard soil with inventive formulations IF.1 or IF.2, respectively, was at least twice as efficient as with the corresponding comparative formulations C-F.3 and C-F.4.

## Claims

1. Formulations comprising at least one compound according to the general formula (I) 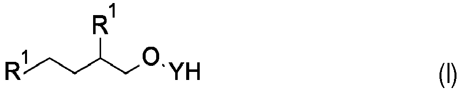
wherein R¹ is a CH(CH₃)₂-group,
YH is (AO)ₙ-H, with
AO being selected from -CH₂CH₂O- and CH₂CH(R²)O- and being different or identical in the case of n > 1
R² being different or identical and selected from C₁-C₁₆-alkyl, branched or linear,
n in the range of from 1 to 30, and
the polydispersity being in the range of from 1.0 to 1.5.

2. Formulations according to claim 1, **characterized in that** said formulation is selected from liquid and solid formulations.

3. Formulations according to claim 1 or 2, **characterized in that** said formulation contains at least one organic complexing agent selected from N,N,N',N'-ethylenediaminetetraacetic acid (EDTA), N,N,N-nitrilotriacetic acid (NTA), 2-phosphono-1,2,4-butanetricarboxylic acid, aminotri(methylenephosphonic acid), 1-hydroxyethylene(1,1-diphosphonic acid), ethylenediaminetetramethylenephosphonic acid, hexamethylenediaminetetramethylenephosphonic acid and diethylenetriaminepentamethylenephosphonic acid and in each case the respective alkali metal salts.

4. Formulations according to any of claims 1 to 3, **characterized in that** the formulation further contains at least one anionic surfactant.

5. Formulations according to any of claims 1 to 4, characterized it comprises at least one organic acid, selected from acetic acid, citric acid, and methanesulfonic acid.

6. Use of a formulation according to any of claims 1 to 5 for hard surface cleaning.

7. Process for making formulations according to any of claims 1 to 5, comprising the step of mixing at least one compound of general formula (I) with water and optionally with at least one organic complexing agent, with at least one anionic surfactants or at least one organic acid, selected from acetic acid, citric acid, and methanesulfonic acid.

8. Compound of general formula (I) 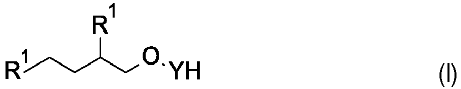
wherein R¹ is a CH(CH₃)₂-group,
YH is (AO)ₙ-H, with
AO being selected from -CH₂CH₂O- and -CH₂CH(R²)O- and being different or identical in the case of n > 1,
R² being different or identical and selected from C₁-C₁₆-alkyl, branched or linear,
n in the range of from 1 to 30,
the polydispersity being in the range of from 1.0 to 1.5.

9. Process for making a compound according to claim 8, **characterized in** reacting the corresponding alcohol according to formula (III) 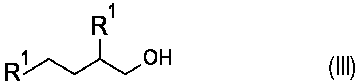 with R¹ being a CH(CH₃)₂-group, with at least one alkylene oxide selected from ethylene oxide and alkylene oxides according to formula (II) 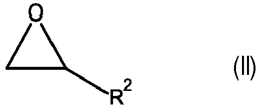 R² being different or identical and selected from C₁-C₁₆-alkyl, branched or linear,
in the presence of at least one catalyst, selected from double-metal cyanides.

10. Process according to claim 9, **characterized in that** alkylene oxide(s) according to formula (II) is/are reacted in a molar excess of at least 1.5 · n, referring to the total of compound according to general formula (III).

11. Process according to claim 9 or 10, **characterized in that** double-metal cyanides are selected from compounds according to general formula (VI)
M¹ₐ[M²(CN)_{b}(A)_{c}]_{d}·f M¹_{g}Xₘ·h(H₂O)·eL·kP (VI),
in which the integers are defined as follows:
M¹ is at least one metal ion chosen from the group consisting of Zn²⁺, Fe²⁺, Fe³⁺, Co³⁺, Ni²⁺, Mn²⁺, Co²⁺, Sn2⁺, Pb²⁺, Mo⁴⁺, Mo⁶⁺, Al³⁺, V⁴⁺, V⁵⁺, Sr²⁺, W⁴⁺, W⁶⁺, Cr²⁺, Cr³⁺, Cd²⁺, Hg²⁺, Pd²⁺, Pt²⁺, V²⁺, Mg²⁺, Ca²⁺, Ba²⁺, Cu²⁺, La³⁺, Ce³⁺, Ce⁴⁺, Eu³⁺, Ti³⁺, Ti⁴⁺, Ag⁺, Rh²⁺, Rh³⁺, Ru²⁺, Ru³⁺,
M² is at least one metal ion chosen from the group consisting of Fe²⁺, Fe³⁺, Co²⁺, Co³⁺, Mn²⁺, Mn³⁺, V⁴⁺, V⁵⁺, Cr²⁺, Cr³⁺, Rh³⁺, Ru²⁺, Ir³⁺,
and selected in a way that M¹ and M² are not identical,
A and X, independently of one another, are anions selected from the group consisting of halide, hydroxide, sulfate, carbonate, cyanide, thiocyanate, isocyanate, cyanate, carboxylate, oxalate, nitrate, nitrosyl, hydrogensulfate, phosphate, dihydrogenphosphate, hydrogenphosphate or hydrogencarbonate,
L is a ligand chosen from the group consisting of alcohols, aldehydes, ketones, ethers, polyethers, esters, polyesters, polycarbonate, ureas, amides, primary, secondary and tertiary amines, ligands with pyridine nitrogen, nitriles, sulfides, phosphides, phosphites, phosphanes, phosphonates and phosphates,
k is an integer greater than or equal to zero, and
P is an organic additive,
a, b, c, d, g and n are chosen such that the electroneutrality of the compound according to general formula (VI) is ensured, where c may be zero,
e is the number of ligand molecules an integer greater than zero, or zero,
f and h, independently of one another, are fractions or integers greater than zero, or zero.

## Patentansprüche

1. Formulierungen, umfassend mindestens eine Verbindung gemäß der allgemeinen Formel (I) 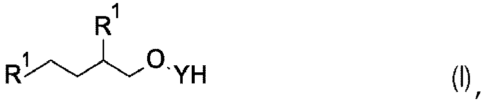
wobei R¹ für eine CH(CH₃)₂-Gruppe steht,
YH für (AO)ₙ-H steht, wobei
AO aus -CH₂CH₂O- und CH₂CH(R²)O- ausgewählt ist und im Fall von n > 1 gleich oder verschieden ist,
R² gleich oder verschieden ist und aus verzweigtem oder linearem C₁-C₁₆-Alkyl ausgewählt ist,
n im Bereich von 1 bis 30 liegt und
die Polydispersität im Bereich von 1,0 bis 1,5 liegt.

2. Formulierungen nach Anspruch 1, **dadurch gekennzeichnet, dass** die Formulierung aus flüssigen und festen Formulierungen ausgewählt ist.

3. Formulierungen nach Anspruch 1 oder 2, **dadurch gekennzeichnet, dass** die Formulierung mindestens einen organischen Komplexbildner, der aus N,N,N',N'-Ethylendiamintetraessigsäure (EDTA), N,N,N-Nitrilotriessigsäure (NTA), 2-Phosphono-1,2,4-Butantricarbonsäure, Aminotri(methylenphosphonsäure), 1-Hydroxyethylen(1,1-diphosphonsäure), Ethylendiamintetramethylenphosphonsäure, Hexamethylendiamintetramethylenphosphonsäure und Diethylentriaminpentamethylenphosphonsäure und in jedem Fall den jeweiligen Alkalimetallsalzen ausgewählt ist, enthält.

4. Formulierungen nach einem der Ansprüche 1 bis 3, **dadurch gekennzeichnet, dass** die Formulierung ferner mindestens ein anionisches Tensid enthält.

5. Formulierungen nach einem der Ansprüche 1 bis 4, **dadurch gekennzeichnet, dass** sie mindestens eine organische Säure, die aus Essigsäure, Citronensäure und Methansulfonsäure ausgewählt ist, umfassen.

6. Verwendung einer Formulierung nach einem der Ansprüche 1 bis 5 zum Reinigen von harten Oberflächen.

7. Verfahren zur Herstellung von Formulierungen nach einem der Ansprüche 1 bis 5, bei dem man mindestens eine Verbindung der allgemeinen Formel (I) mit Wasser und gegebenenfalls mit mindestens einem organischen Komplexbildner, mit mindestens einem anionischen Tensid oder mindestens einer organischen Säure, die aus Essigsäure, Citronensäure und Methansulfonsäure ausgewählt ist, mischt.

8. Verbindung der allgemeinen Formel (I) 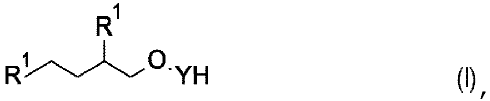
wobei R¹ für eine CH(CH₃)₂-Gruppe steht,
YH für (AO)ₙ-H steht, wobei
AO aus -CH₂CH₂O- und -CH₂CH(R²)O- ausgewählt ist und im Fall von n > 1 gleich oder verschieden ist,
R² gleich oder verschieden ist und aus verzweigtem oder linearem C₁-C₁₆-Alkyl ausgewählt ist,
n im Bereich von 1 bis 30 liegt und
die Polydispersität im Bereich von 1,0 bis 1,5 liegt.

9. Verfahren zur Herstellung einer Verbindung nach Anspruch 8, **dadurch gekennzeichnet, dass** man den entsprechenden Alkohol gemäß Formel (III) 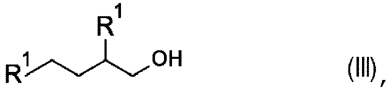 wobei R¹ für eine CH(CH₃)₂-Gruppe steht,
in Gegenwart mindestens eines Katalysators, der aus Doppelmetallcyaniden ausgewählt wird, mit mindestens einem Alkylenoxid, das aus Ethylenoxid und Alkylenoxiden gemäß Formel (II) 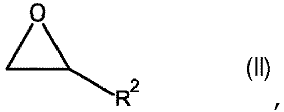 wobei R² gleich oder verschieden ist und aus verzweigtem oder linearem C₁-C₁₆-Alkyl ausgewählt ist,
ausgewählt wird, umsetzt.

10. Verfahren nach Anspruch 9, **dadurch gekennzeichnet, dass** das Alkylenoxid bzw. die Alkylenoxide gemäß Formel (II) in einem molaren Überschuss von mindestens 1,5·n, bezogen auf die Gesamtmenge der Verbindung gemäsß der allgemeinen Formel (III), umgesetzt wird bzw. werden.

11. Verfahren nach Anspruch 9 oder 10, **dadurch gekennzeichnet, dass** die Doppelmetallcyanide aus Verbindungen gemäß der allgemeinen Formel (VI)
M¹ₐ[M²(CN)_{b}(A)_{c}]_{d}·fM¹_{g}Xₘ·h(H₂O)·eL·kP (VI)
ausgewählt werden, wobei die Variablen wie folgt definiert sind:
M¹ ist mindestens ein Metallion, ausgewählt aus der Gruppe bestehend aus Zn²⁺, Fe²⁺, Fe³⁺, Co³⁺, Ni²⁺, Mn²⁺, Co²⁺, Sn²⁺, Pb²⁺, Mo⁴⁺, Mo⁶⁺, Al³⁺, V⁴⁺, V⁵⁺, Sr²⁺, W⁴⁺, W⁶⁺, Cr²⁺, Cr³⁺, Cd²⁺, Hg²⁺, Pd²⁺, Pt²⁺, V²⁺, Mg²⁺, Ca²⁺ Ba²⁺ Cu²⁺ La³⁺, Ce³⁺, Ce⁴⁺, Eu³⁺, Ti³⁺, Ti⁴⁺, Ag⁺, Rh²⁺, Rh³⁺, Ru²⁺, Ru³⁺,
M² ist mindestens ein Metallion, ausgewählt aus der Gruppe bestehend aus Fe²⁺, Fe³⁺, Co²⁺, Co³⁺, Mn²⁺, Mn³⁺, V⁴⁺, V⁵⁺, Cr²⁺, Cr³⁺, Rh³⁺, Ru²⁺, Ir³⁺,
und derart ausgewählt, dass M¹ und M² nicht gleich sind,
A und X sind unabhängig voneinander Anionen, ausgewählt aus der Gruppe bestehend aus Halogenid, Hydroxid, Sulfat, Carbonat, Cyanid, Thiocyanat, Isocyanat, Cyanat, Carboxylat, Oxalat, Nitrat, Nitrosyl, Hydrogensulfat, Phosphat, Dihydrogenphosphat, Hydrogenphosphat oder Hydrogencarbonat,
L ist ein Ligand, ausgewählt aus der Gruppe bestehend aus Alkoholen, Aldehyden, Ketonen, Ethern, Polyethern, Estern, Polyestern, Polycarbonat, Harnstoffen, Amiden, primären, sekundären und tertiären Aminen, Liganden mit Pyridin-Stickstoff, Nitrilen, Sulfiden, Phosphiden, Phosphiten, Phosphanen, Phosphonaten und Phosphaten,
k ist eine ganze Zahl größer oder gleich Null, und P ist ein organischer Zusatzstoff,
a, b, c, d, g und n sind so ausgewählt, dass die Elektroneutralität der Verbindung gemäß der allgemeinen Formel (VI) gewährleistet ist, wobei c = 0 sein kann,
e ist die Anzahl der Ligandenmoleküle, eine ganze Zahl größer Null oder Null,
f und h sind unabhängig voneinander eine gebrochene oder ganze Zahl größer Null oder Null.

## Revendications

1. Formulations comprenant au moins un composé selon la formule générale (I) 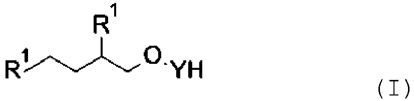
dans laquelle R¹ est un groupe CH(CH₃)₂,
YH est (AO)ₙ-H, avec
AO étant choisi parmi -CH₂CH₂O- et CH₂CH(R²)O- et étant différent ou identique dans le cas de n > 1
R² étant différent ou identique et choisi parmi alkyle en C₁-C₁₆, ramifié ou linéaire,
n étant dans la plage de 1 à 30, et
la polydispersité étant dans la plage de 1,0 à 1,5.

2. Formulations selon la revendication 1, **caractérisées en ce que** lesdites formulations sont choisies parmi des formulations liquides et solides.

3. Formulations selon la revendication 1 ou 2, **caractérisées en ce que** lesdites formulations contiennent au moins un agent complexant organique choisi parmi l'acide N,N,N',N'-éthylènediaminetétraacétique (EDTA), l'acide N,N,N-nitrilotriacétique (NTA), l'acide 2-phosphono-1,2,4-butanetricarboxylique, l'acide aminotri(méthylènephosphonique), l'acide 1-hydroxyéthylène(1,1-diphosphonique), l'acide éthylènediaminetétraméthylènephosphonique, l'acide hexaméthylènediaminetétraméthylènephosphonique et l'acide diéthylènetriaminepentaméthylènephosphonique et dans chaque cas les sels de métal alcalin respectifs.

4. Formulations selon l'une quelconque des revendications 1 à 3, **caractérisées en ce que** les formulations contiennent en outre au moins un tensioactif anionique.

5. Formulations selon l'une quelconque des revendications 1 à 4, **caractérisées en ce qu'**elles comprennent au moins un acide organique, choisi parmi l'acide acétique, l'acide citrique et l'acide méthanesulfonique.

6. Utilisation d'une formulation selon l'une quelconque des revendications 1 à 5 pour le nettoyage de surface dure.

7. Procédé de fabrication de formulations selon l'une quelconque des revendications 1 à 5, comprenant l'étape de mélange d'au moins un composé de formule générale (I) avec de l'eau et facultativement avec au moins un agent complexant organique, avec au moins un tensioactif anionique ou au moins un acide organique, choisi parmi l'acide acétique, l'acide citrique et l'acide méthanesulfonique.

8. Composé de formule générale (I) 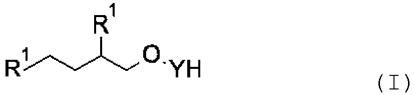
dans laquelle R¹ est un groupe CH(CH₃)₂,
YH est (AO)ₙ-H, avec
AO étant choisi parmi -CH₂CH₂O- et -CH₂CH(R²)O- et étant différent ou identique dans le cas de n > 1,
R² étant différent ou identique et choisi parmi alkyle en C₁-C₁₆, ramifié ou linéaire,
n étant dans la plage de 1 à 30,
la polydispersité étant dans la plage de 1,0 à 1,5.

9. Procédé de fabrication d'un composé selon la revendication 8, **caractérisé par** la réaction de l'alcool correspondant selon la formule (III) 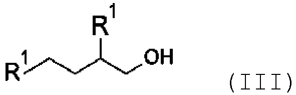 R¹ étant un groupe CH(CH₃)₂-,
avec au moins un oxyde d'alkylène choisi parmi l'oxyde d'éthylène et des oxydes d'alkylène selon la formule (II) 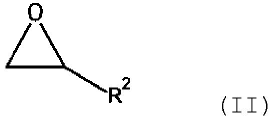 R² étant différent ou identique et choisi parmi alkyle en C₁-C₁₆, ramifié ou linéaire, en présence d'au moins un catalyseur, choisi parmi des cyanures à double métal.

10. Procédé selon la revendication 9, **caractérisé en ce qu'**un ou plusieurs oxyde(s) d'alkylène selon la formule (II) réagit/réagissent en un excès molaire d'au moins 1,5 par rapport au total du composé selon la formule générale (III).

11. Procédé selon la revendication 9 ou 10, **caractérisé en ce que** les cyanures à double métal sont choisis parmi des composés selon la formule générale (VI)
M¹ₐ[M²(CN)_{b}(A)_{c}]_{d}.fM¹_{g}Xₘ.h(H₂O).eL.kP (VI),
dans laquelle les entiers sont définis comme suit :
M¹ est au moins un ion métallique choisi dans le groupe constitué de Zn²⁺, Fe²⁺, Fe³⁺, Co³⁺, Ni²⁺, Mn²⁺, Co²⁺, Sn²⁺, Pb²⁺, Mo⁴⁺, Mo⁶⁺, Al³⁺, V⁴⁺, V⁵⁺, Sr²⁺, W⁴⁺, W⁶⁺, Cr²⁺, Cr³⁺, Cd²⁺, Hg²⁺, Pd²⁺, Pt²⁺, V²⁺, Mg²⁺, Ca²⁺, Ba²⁺, Cu²⁺, La³⁺, Ce³⁺, Ce⁴⁺, Eu³⁺, Ti³⁺, Ti⁴⁺, Ag⁺, Rh²⁺, Rh³⁺, Ru²⁺, Ru³⁺,
M² est au moins un ion métallique choisi dans le groupe constitué de Fe²⁺, Fe³⁺, Co²⁺, Co³⁺, Mn²⁺, Mn³⁺, V⁴⁺, V⁵⁺, Cr²⁺, Cr³⁺, Rh³⁺, Ru²⁺, Ir³⁺,
et choisis de telle manière que M¹ et M² ne soient pas identiques,
A et X, indépendamment l'un de l'autre, sont des anions choisis dans le groupe constitué d'halogénure, hydroxyde, sulfate, carbonate, cyanure, thiocyanate, isocyanate, cyanate, carboxylate, oxalate, nitrate, nitrosyle, hydrogénosulfate, phosphate, dihydrogénophosphate, hydrogénophosphate ou hydrogénocarbonate,
L est un ligand choisi dans le groupe constitué d'alcools, aldéhydes, cétones, éthers, polyéthers, esters, polyesters, polycarbonate, urées, amides, amines primaires, secondaires et tertiaires, ligands avec l'azote de pyridine, nitriles, sulfures, phosphures, phosphites, phosphanes, phosphonates et phosphates,
k est un entier supérieur ou égal à zéro, et P est un additif organique,
a, b, c, d, g et n sont choisis de sorte que l'électroneutralité du composé selon la formule générale (VI) soit assurée, où c peut être zéro,
e est le nombre de molécules de ligand et un entier supérieur à zéro, ou zéro,
f et h, indépendamment l'un de l'autre, sont des fractions ou des entiers supérieurs à zéro, ou zéro.
